# EUROPÄISCHE PATENTANMELDUNG

(11) **EP 1 287 811 A2**
(43) Veröffentlichungstag der Anmeldung: **05.03.2003**
(21) Anmeldenummer: 02018560.9
(22) Anmeldetag: 17.08.2002
(51) Int. Cl.: A61K 7/06

(54) **Milchsäureester in Haarkuren**

(30) Priorität: 30.08.2001 DE 10142136
(71) Anmelder: Beiersdorf Aktiengesellschaft, 20245 Hamburg (DE)
(72) Erfinder: Argembeaux, Horst, 21465 Wentorf (DE); Demitz, Michael, 22529 Hamburg (DE)

(57) **Zusammenfassung**

Verwendung von Milchsäureestern in Haarkonditionierern und Haarkuren, die im Haar verbleiben, zur Verbesserung der Verteilbarkeit derselben im Haar sowie zur Verbesserung des Griffempfindens des Haares.

## Beschreibung

Die vorliegende Erfindung betrifft Milchsäureester in Haarkuren und Haarkonditioniermitteln.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluss des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Ein Ziel der Haarpflege ist es, Kopfhaut und -haar zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl (guter "Griff") gelten als Merkmale für natürliches, gesundes Haar.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche und auch fettlösliche Haarbestandteile durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Diese können so formuliert werden, dass sie nicht nur der Pflege und des Schutzes des einzelnen Haars dienen, sondern auch das Erscheinungsbild der Haartracht insgesamt verbessern, beispielsweise dadurch, dass sie das Haar geschmeidig und somit frisierwillig machen, ihm einen angenehmen Griff geben und es seidig glänzend machen. Solche Zubereitungen werden gemeinhin auch als Haarkonditionierer (engl. Conditioner) oder Haarkuren bezeichnet.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, dass sie nicht nur der Pflege des einzelnen Haars dienen, sondern auch das Aussehen einer Frisur insgesamt verbessern, beispielsweise dadurch, dass sie dem Haar mehr Fülle verleihen, die Frisur über einen längeren Zeitraum fixieren oder die Frisierbarkeit verbessern.
Haarkuren enthalten als Hauptwirkstoffe sogenannte Haarkonditioniermittel. Haarkonditioniermittel sind Zusätze zu Haarpflegemitteln, welche die Kämmbarkeit des nassen und des getrockneten Haares verbessern, die statische Aufladung des Haares neutralisieren und zugleich das Aussehen, die Griffigkeit, die Fülle, den Glanz und insgesamt das Frisiervermögen des Haares begünstigen. Die Wirkung eines Haarkonditioniermittels ist weitgehend von dem Aufziehvermögen der Substanz auf die Haaroberfläche abhängig.

Zu den Haarkonditioniermitteln zählen insbesondere die quaternären Ammoniumverbindungen, die verschiedenen Protein-Hydrolysate, ferner aber auch verschiedene Fette oder fettähnliche Produkte, z..B. die Silicone und die Silikon-Derivate, Glykole und nach neueren Untersuchungen auch kationische Polymere.

Trotz der Vielzahl der bekannten und vorgeschlagenen Formulierungen sind die bekannten Haarkuren nach wie vor verbesserungsfähig, insbesondere ihre Verteilbarkeit im Haar und der Griff des Haares nach Anwendung der Formulierung. Dieses betrifft in besonderem Maße Kuren die im Haar verbleiben.

Es war daher die Aufgabe der vorliegenden Erfindung, die Nachteile des Standes der Technik zu beseitigen und Haarkuren zu entwickeln, die sich durch eine gute Verteilbarkeit im Haar und nach der Anwendung durch einen besonders guten Griff des Haares auszeichnen.

Vollkommen überraschend und für den Fachmann nicht vorhersehbar wird die Aufgabe gelöst durch die Verwendung von Milchsäureestern in Haarkuren. Dabei haben sich C₁₂ - bis C₁₃ - Alkyllactate als erfindungsgemäß besonders vorteilhaft herausgestellt. Zwar beschreibt die EP 0 878 188 die Verwendung von Milchsäureestern in Haarpflegemitteln, doch konnte diese Schrift nicht den Weg zur vorliegenden Erfindung weisen, da die Milchsäureester in diesem Fall nur als Rückfettungsmittel verwendet werden.

Erfindungsgemäß vorteilhaft ist dabei eine Konzentration von 0,5 bis 2,5 Gew.-% und insbesondere 1 Gew.-% Milchsäureester, bezogen auf das Gesamtgewicht der Formulierung.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiele** | | | | |
|---|---|---|---|---|
| Produktbezeichnung | Menge/% | Menge/% | Menge/% | Menge/% |
| Laureth-4 | 0,6 | 0,4 | 0,5 | 0.5 |
| Cocosnußfettsäureethylhex ylester | 0,9 | 0,8 | 0,8 | 1 |
| C12-13 Alkyllactat | 0,5 | 0,7 | 2,5 | 1 |
| Acrylate/C10-30 Alkyl Acrylat-Crosspolymer | 0,9 | 0,7 | 1,0 | 0.8 |
| Hydroxypropyl Guarhydroxypropyltrimoniu mchlorid | 0,1 | 0,3 | 0,2 | 0.2 |
| PVPNA Copolymer | 4 | 4 | 3 | 5 |
| Propylenglycol | 3 | 5 | 6 | 7 |
| Palmitamidopropyltrimoniu mchlorid | 0,4 | 0,3 | 0,5 | 0.5 |
| Parfum, Lösungsvermittler, Konservierung, Komlexierungsmittel, Puffer | 2 | 2 | 2 | 2 |
| Aqua | Ad. 100 | Ad. 100 | Ad. 100 | Ad. 100 |

## Patentansprüche

1. Verwendung von Milchsäureestern in Haarkonditionierern und Haarkuren zur leichteren Verteilbarkeit derselben im Haar.

2. Verwendung von Milchsäureestern in Haarkonditionierern und Haarkuren zur Verbesserung des Griffempfindens des Haares.

3. Verwendung von Milchsäureestern in Haarkonditionierern und Haarkuren, die im Haar verbleiben, zur leichteren Verteilbarkeit derselben im Haar.

4. Verwendung von Milchsäureestern in Haarkonditionierern und Haarkuren, die im Haar verbleiben, zur Verbesserung des Griffempfindens des Haares.

5. Verwendung nach Anspruch 1 oder 2, **dadurch gekennzeichnet dass** es sich beim Milchsäureester um C₁₂ - bis C₁₃ - Alkyllactate handelt.

6. Verwendung nach einem der vorhergehenden Ansprüche, **dadurch gekennzeichnet dass** der Milchsäureester in einer Konzentraion von 0,5 bis 2,5 Gewichts-% bezogen auf das Gesamtgewicht der Formulierung eingesetzt wird.
